# EUROPÄISCHE PATENTSCHRIFT

(11) **EP 3 843 691 B1**
(45) Veröffentlichungstag und Bekanntmachung des Hinweises auf die Patenterteilung: **18.06.2025**
(21) Anmeldenummer: 19736362.5
(22) Anmeldetag: 03.07.2019
(51) Int. Cl.: A61K 8/06, A61K 8/34, A61K 8/37, A61K 8/39, A61K 8/46, A61K 8/49, A61K 8/55, A61K 8/73, A61Q 19/00

(54) **ACRYLATFREIE KOSMETISCHE EMULSION**
ACRYLATE-FREE COSMETIC EMULSION
ÉMULSION COSMÉTIQUE SANS ACRYLATE

(30) Priorität: 28.08.2018 DE 102018214478
(43) Veröffentlichungstag der Anmeldung: 07.07.2021
(73) Patentinhaber: Beiersdorf AG, 22529 Hamburg (DE)
(72) Erfinder: RASCHKE, Thomas, 25421 Pinneberg (DE); PIRADASHVILI, Keti, 22111 Hamburg (DE)
(74) Vertreter: Beiersdorf AG
(86) Internationale Anmeldenummer: PCT/EP2019/067803
(87) Internationale Veröffentlichungsnummer: WO 2020/043365

(56) Entgegenhaltungen:
- CN-B- 105 434 307
- DATABASE GNPD [online] MINTEL; 13 March 2013 (2013-03-13), ANONYMOUS: "Cleanser", XP055615692, retrieved from https://www.gnpd.com/sinatra/recordpage/2016848/ Database accession no. 2016848
- DATABASE GNPD [online] MINTEL; 21 September 2011 (2011-09-21), ANONYMOUS: "After Sun Repair Lotion", XP055615689, retrieved from https://www.gnpd.com/sinatra/recordpage/1625513/ Database accession no. 1625513
- DATABASE GNPD [online] MINTEL; 20 October 2017 (2017-10-20), ANONYMOUS: "Rejuvenating Face Wash", XP055615688, retrieved from https://www.gnpd.com/sinatra/recordpage/5178271/ Database accession no. 5178271
- DATABASE GNPD [online] MINTEL; 20 October 2017 (2017-10-20), ANONYMOUS: "Rejuvenating Face Wash", XP055615688, retrieved from www.gnpd.com Database accession no. 5178271
- DATABASE GNPD [online] MINTEL; 21 September 2011 (2011-09-21), ANONYMOUS: "After Sun Repair Lotion", XP055615689, retrieved from www.gnpd.com Database accession no. 1625513
- DATABASE GNPD [online] MINTEL; 13 March 2013 (2013-03-13), ANONYMOUS: "Cleanser", XP055615692, retrieved from www.gnpd.com Database accession no. 2016848

## Beschreibung

Kosmetische Produkte dienen im Allgemeinen nicht nur dazu schön und attraktiv auszusehen, sondern sie tragen mit ihrer Wirkung entscheidend zu einem gesteigerten Selbstwertgefühl und zum Wohlbefinden der Menschen bei. Dementsprechend werden die verschiedensten kosmetischen Produkte zur täglichen Reinigung und Pflege der menschlichen Haut eingesetzt.

Hautpflegeprodukte bestehen in der Regel aus Emulsionen. Unter Emulsionen versteht man im Allgemeinen heterogene Systeme, die aus zwei nicht oder nur begrenzt miteinander mischbaren Flüssigkeiten bestehen, die üblicherweise als Phasen bezeichnet werden und bei denen eine der beiden Flüssigkeiten in Form feinster Tröpfchen in der anderen Flüssigkeit dispergiert ist. Äußerlich und mit bloßem Auge betrachtet erscheinen Emulsionen homogen.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter einer O/W-Emulsion ist durch das Wasser geprägt. Bei einer Wasser-in-Öl-Emulsion (W/O-Emulsion, z. B. Butter) handelt es sich um das umgekehrte Prinzip, wobei der Grundcharakter hier durch das Öl bestimmt wird.

Zur Stabilisierung und zur Verdickung von O/W-Emulsionen werden zumeist Acrylat-basiete Polymere in diese Formulierungen eingearbeitet. Zu den Acrylat-basierten Polymeren zählen Polymere, welche aus Homo- oder Copolymerisation mit Acryl- und/oder Methacrylsäure erhalten werden. Beispiele hierfür sind u.a. Carbomer oder Acrylate Copolymer.

Der Stand der Technik kennt unter anderem die Dokumente DE 10148825 A1, DE 19938756 A1 und DE 29924371 U1, welche pflegende O/W-Emulsionen mit Acrylat-basierten Polymeren offenbaren. Jedoch ist der Einsatz dieser Acrylat-basierten Polymere zunehmend in der Kritik, da deren biologische Abbaubarkeit nicht vollständig geklärt ist.

Im Gegensatz zu den Acrylat-basierten Polymeren werden Polymere auf Basis von Polysacchariden aus nachwachsenden Rohstoffen gewonnen, so dass sich diese unproblematisch biologisch abbauen lassen und zudem oftmals preisgünstig sind.

Problematisch ist jedoch der Umstand, dass ein einfacher Austausch von Acrylat-basierten Polymeren gegen Polymere auf Basis von Polysacchariden oftmals nicht möglich ist, da diese zweiphasigen Formulierungen zumeist nicht die nötige Langzeitstabilität aufweisen und es so zu einer ungewünschten Phasentrennung der O/W-Emulsion kommt. Dieses tritt insbesondere auf, wenn die Öl-Phase der Emulsion in einem Anteil von mehr als 3 Gew.-%, bevorzugt mehr als 5 Gew.-%, bezogen auf das Gesamtgewicht der Emulsion, vorliegt. Weiterhin ist der Umstand problematisch, dass bei Einsatz von Polymeren auf Basis von Polysacchariden, die Formulierungen beim Auftragen auf der Haut unzureichend schnell einziehen und oftmals signifikante Mengen weiße Rückstände hinterlassen. Somit werden derartige Formulierungen vom Verbraucher oftmals als nicht angenehm bezeichnet. Stattdessen wünscht sich der Verbraucher Formulierungen, welche schnell und ohne Rückstände einziehen, somit eine leichte Textur aufweisen. Somit führt ein einfacher Austausch von Acrylat-basierten Polymeren gegen Polymere auf Basis von Polysacchariden zu erheblichen Produktnachteilen.

Der Stand der Technik kennt weiterhin die Produkte mit der Mintel GNPD Eintragungsnummer 5178271 (Rejuvenating Face Wash), 1625513 (After Sun Repair Lotion) und 2016848 (Cleanser- Anti Aging Collagen Plus). Keines dieser Produkte konnte einen Hinweis auf die Erfindung geben.

Weiterhin ist das Dokument CN 105434307 B, welches ein Antiakneprodukt und die Herstellungsverfahren davon betrifft. Ein Hinweis auf die vorliegende Erfindung wird nicht gegeben.

Aufgabe der vorliegenden Erfindung war es daher eine acrylatfreie kosmetische O/W-Emulsion bereitzustellen, welche die Nachteile des Standes der Technik nicht aufweist. Insbesondere war es Aufgabe eine O/W-Emulsion mit Polymeren auf Basis von Polysacchariden bereitzustellen, welche temperaturstabiler sind. Zusätzlich war es eine Aufgabe O/W-Emulsionen bereitzustellen, welche beim Auftragen schnell einziehen sowie geringere Mengen an Rückständen aufweisen.

Überraschend gelöst wird/werden die Aufgaben durch eine acrylatfreie kosmetische O/W-Emulsion enthaltend
a) mindestens eine Substanz gewählt aus der Gruppe der Alkalimetallalkylsulfate und der Alkalimetallalkylphosphate, wobei die Alkylgruppe jeweils 16 bis 18 Kohlenstoffatome aufweist,
b) Hydroxypropylstärkephosphat, und
c) Glycerinmonostearat und/oder Sorbitanmonostearat.

Gegenstand der Erfindung ist auch die Verwendung eine Mischung aus
a) mindestens eine Substanz gewählt aus der Gruppe der Alkalimetallalkylsulfate und der Alkalimetallalkylphosphate, wobei die Alkylgruppe jeweils 16 bis 18 Kohlenstoffatome aufweist,
b) Hydroxypropylstärkephosphat, und
c) Glycerinmonostearat und/oder Sorbitanmonostearat
zur Verhinderung einer Phasentrennung in einer acrylatfreien O/W-Emulsion.

Sollten nachfolgend Gewichtsprozentangaben (Gew.-%) ohne Bezugnahme auf eine bestimmte Zusammensetzung oder spezifische Mischung angegeben werden, so beziehen sich diese Angaben immer auf das Gesamtgewicht der kosmetischen O/W-Emulsion. Sollten nachfolgend Verhältnisse von Komponenten/Substanzen/Stoffgruppen offenbart werden, so beziehen sich diese Verhältnisse auf Gewichtsverhältnisse der genannten Komponenten/ Substanzen/Stoffgruppen.

Werden nachfolgend Gewichtsprozentbereiche für die Bestandteile der kosmetischen O/W-Emulsion angegeben, so umfasst die Offenbarung der vorliegenden Anmeldung ebenfalls alle Einzelwerte in Schritten von 0,1 Gew.-% innerhalb dieser Gewichtsprozentbereiche.

Die Formulierungen "erfindungsgemäß", "erfindungsgemäß vorteilhaft", "vorteilhaft im Sinne der Vorliegenden Erfindung" etc. beziehen sich im Rahmen der vorliegenden Offenbarung immer sowohl auf die erfindungsgemäße Zubereitung sowie die erfindungsgemäße Verwendung.

Sofern nicht anders angegeben wurden alle Versuche unter Normalbedingungen durchgeführt. Der Begriff "Normalbedingungen" bedeutet 20°C, 1013 hPa und eine relative Luftfeuchtigkeit von 50%.

Wird der Begriff Haut verwendet, so bezieht dieser sich bevorzugt auf die menschliche Haut.

Die erfindungsgemäße O/W-Emulsion ist acrylatfrei. Acrylatfrei bedeutet im Sinne der vorliegenden Erfindung, dass der Gesamtanteil an Acrylat-basierten Polymeren weniger als 0,2 Gew.-%, bevorzugt weniger als 0,1 Gew.-% und weiterhin bevorzugt weniger 0,05 Gew.-% und insbesondere bevorzugt 0 Gew-% beträgt, wobei sich die Angaben auf das Gesamtgewicht der Emulsion beziehen. Erfindungsgemäß werden unter Acrylat-basierten Polymeren alle Polymere verstanden, welche aus einer Homo- oder Copolymerisation mit Acryl- und/oder Methacrylsäure gewonnen werden.

Erfindungsgemäß umfasst die kosmetische O/W-Emulsion eine Substanz gewählt aus der Gruppe der Alkalimetallalkylsulfate und der Alkalimetallalkylphosphate, wobei die Alkylgruppe jeweils 16 bis 18 Kohlenstoffatome aufweist.

Unter den Alkalimetallalkylsulfaten werden bevorzugt Natriumcetylsulfat, Natriumstearylsulfat und deren Mischung bekannt unter dem Namen Natriumcetearylsulfat eingesetzt.

Weiterhin wird bevorzugt unter den Alkalimetallalkylphosphaten Kaliumcetylphosphat eingesetzt.

Ein bevorzugter Gegenstand der Erfindung ist somit eine acrylatfreie kosmetische O/W-Emulsion enthaltend,
a) mindestens eine Substanz gewählt aus der Gruppe-Natriumcetylsulfat, Natriumstearylsulfat, Natriumcetearylsulfat und Kaliumcetylphosphat,
b) Hydroxypropylstärkephosphat, und
c) Glycerinmonostearat und/oder Sorbitanmonostearat.

Die Substanzen der Gruppe der Alkalimetallalkylsulfate, der Alkalimetallalkylphosphate und Natriumstearoylglutamat können erfindungsgemäß als Mischung oder als Einzelkomponente eingesetzt werden.

Ein weiter bevorzugter Gegenstand der Erfindung ist eine acrylatfreie kosmetische O/W-Emulsion enthaltend,
a) Natriumcetylsulfat, Natriumstearylsulfat und/oder Natriumcetearylsulfat,
b) Hydroxypropylstärkephosphat, und
c) Glycerinmonostearat und/oder Sorbitanmonostearat.

Ein weiterer bevorzugter Gegenstand der Erfindung ist eine acrylatfreie kosmetische O/W-Emulsion enthaltend,
a) mindestens eine Substanz gewählt aus der Gruppe der Alkalimetallalkylphosphate, dadurch gekennzeichnet, dass die Alkylgruppe jeweils 16 bis 18 Kohlenstoffatome aufweist, wobei die Substanz insbesondere bevorzugt Kaliumcetylphosphat ist,
b) Hydroxypropylstärkephosphat, und
c) Glycerinmonostearat und/oder Sorbitanmonostearat.

Es ist erfindungsgemäß weiterhin vorteilhaft, wenn die jeweils vorstehend unter a) genannten Substanzen in einem Gesamtanteil von 0,01 Gew.-% bis 5,0 Gew.-%, mehr bevorzugt von 0,05 Gew.-% bis 2,0 Gew.-% und am meisten bevorzugt von 0,1 Gew.-% bis 1,5 Gew.-% enthalten sind, bezogen auf das Gesamtgewicht der Emulsion.

Weiterhin ist es vorteilhaft im Sinne der vorliegenden Erfindung, wenn der Anteil von Hydroxypropylstärkephosphat von 0,1 Gew.-% bis 10 Gew.-%, bevorzugt von 0,2 Gew.-% bis 5,0 Gew.-% und insbesondere bevorzugt von 0,5 Gew.-% bis 3 Gew.-%, bezogen auf das Gesamtgewicht der Emulsion, beträgt.

Generell ist Hydroxypropylstärkephosphat ein Veresterungsprodukt basierend auf Stärke. Erfindungsgemäß ist es möglich Hydroxypropylstärkephosphat basierend auf verschiedenen Stärken einzusetzen. Dem Fachmann sind unter anderem Weizen oder Kartoffelstärke bekannt.

Überraschend hat sich allerdings für den Fachmann herausgestellt, dass, wenn die eingesetzte Hydroxypropylstärkephosphat ein Veresterungsprodukt basierend auf Maisstärke ist, die erfindungsgemäße O/W-Emulsion deutlich weniger klebrig auf der Haut ist, als wenn andere Stärkequellen verwendet werden. Vorteilhafte Ausführungsformen der vorliegenden Erfindung sind somit dadurch gekennzeichnet, dass das eingesetzte Hydroxypropylstärkephosphat ein Veresterungsprodukt basierend auf Maisstärke ist. Innerhalb dieser Ausführungsformen ist es weiter bevorzugt, wenn der Anteil des Hydroxypropylstärkephosphat, welches ein Veresterungsprodukt basierend auf Maisstärke ist, von 0,1 Gew.-% bis 10 Gew.-%, bevorzugt von 0,2 Gew.-% bis 5,0 Gew.-% und insbesondere bevorzugt von 0,5 Gew.-% bis 3 Gew.-%, bezogen auf das Gesamtgewicht der Emulsion, beträgt.

Ein erfindungsgemäßes Hydroxypropylstärkephosphat basierend auf Maisstärke ist unter dem Handelsnamen C*HiForm A 12747 von Cargill zu beziehen.

Ferner ist es erfindungsgemäß vorteilhaft, wenn der Gesamtanteil von Glycerinmonostearat und/oder Sorbitanmonostearat von 0,1 Gew.-% bis 5,0 Gew.-%, bevorzugt 0,2 Gew.-% bis 3,0 Gew.-% und insbesondere bevorzugt von 0,5 Gew.-% bis 2,5 Gew.-%,beträgt, wobei sich die Angaben auf das Gesamtgewicht der Emulsion beziehen.

Weitere vorteilhafte erfindungsgemäße O/W-Emulsionen enthalten Glycerinmonostearat in einem Anteil von 0,1 Gew.-% bis 5,0 Gew.-%, bevorzugt 0,2 Gew.-% bis 3,0 Gew.-% und insbesondere bevorzugt von 0,5 Gew.-% bis 2,5 Gew.-%,beträgt, wobei sich die Angaben auf das Gesamtgewicht der Emulsion beziehen und wobei vorteilhaft kein Sorbitanmonostearat enthalten ist.

Weitere vorteilhafte erfindungsgemäße O/W-Emulsionen enthalten Sorbitanmonostearat in einem Anteil von 0,1 Gew.-% bis 5,0 Gew.-%, bevorzugt 0,2 Gew.-% bis 3,0 Gew.-% und insbesondere bevorzugt von 0,5 Gew.-% bis 2,5 Gew.-%,beträgt, wobei sich die Angaben auf das Gesamtgewicht der Emulsion beziehen und wobei vorteilhaft kein Glycerinmonostearate enthalten ist.

Weiterhin wurde überraschend gefunden, dass sich insbesondere Emulsionen mit der erfindungsgemäßen Kombination stabilisieren lassen, welche dadurch gekennzeichnet sind, dass der Anteil der Ölphase der Emulsion von mehr als 3 Gew.-% bis 30 Gew.-%, bevorzugt von mehr als 4 Gew.-% bis 28 Gew.-% und insbesondere bevorzugt von mehr als 5 Gew.-% bis 25 Gew.-%, bezogen auf das Gesamtgewicht der Emulsion beträgt. Somit lassen sich überraschender Weise Emulsionen mit einem großen Ölphasen-Anteil stabilisieren.

Erfindungsgemäß zählen Emulgatoren und Tenside nicht zur Ölphase. Das heißt, dass unter anderem Glycerinmonostearat, Natriumalkylsulfate, Kaliumcetylphosphat, Glycerinmonostearat und Sorbitanmonostearat nicht zur Ölphase zählen.

Unter Emulgatoren werden alle Substanzen verstanden, welche im International Cosmetic Ingredient Dictionary and Handbook, Thirteenth Edition 2010, (ISBN 1-882621-47-6) unter der Bezeichnung "emulsifying agent" geführt werden. Unter Tensiden werden alle Substanzen verstanden, welche im International Cosmetic Ingredient Dictionary and Handbook, Thirteenth Edition 2010, (ISBN 1-882621-47-6) unter der Bezeichnung " surfactant " geführt werden.

Eine bevorzugte Ausführungsform der Erfindung ist dadurch gekennzeichnet, dass die O/W-Emulsion, bezogen auf das Gesamtgewicht der Emulsion,
a) 0,1 Gew.-% bis 1,0 Gew.-% mindestens eine Substanz gewählt aus der Gruppe der Alkalimetallalkylsulfate und der Alkalimetallalkylphosphate, wobei die Alkylgruppe jeweils 16 bis 18 Kohlenstoffatome aufweist,
b) 0,5 Gew.-% bis 3,0 Gew.-% Hydroxypropylstärkephosphat, und
c) 0,5 Gew.-% bis 2,5 Gew.-% Glycerinmonostearat und/oder Sorbitanmonostearat, enthält und der Anteil der Ölphase der Emulsion von mehr als 5 Gew.-% bis 25 Gew.-% beträgt.

Eine weitere bevorzugte Ausführungsform der Erfindung ist dadurch gekennzeichnet, dass die O/W-Emulsion, bezogen auf das Gesamtgewicht der Emulsion,
a) 0,1 Gew.-% bis 1,0 Gew.-% mindestens eine Substanz gewählt aus der Gruppe der Alkalimetallalkylsulfate und der Alkalimetallalkylphosphate, wobei die Alkylgruppe jeweils 16 bis 18 Kohlenstoffatome aufweist,
b) 0,5 Gew.-% bis 3,0 Gew.-% Hydroxypropylstärkephosphat, und
c) 0,5 Gew.-% bis 2,5 Gew.-% Glycerinmonostearat,
enthält und der Anteil der Ölphase der Emulsion von mehr als 5 Gew.-% bis 25 Gew.-% beträgt.

Eine bevorzugte Ausführungsform der Erfindung ist dadurch gekennzeichnet, dass die O/W-Emulsion, bezogen auf das Gesamtgewicht der Emulsion,
a) 0,1 Gew.-% bis 1,0 Gew.-% mindestens eine Substanz gewählt aus der Gruppe-der Alkalimetallalkylsulfate und der Alkalimetallalkylphosphate, wobei die Alkylgruppe jeweils 16 bis 18 Kohlenstoffatome aufweist,
b) 0,5 Gew.-% bis 3,0 Gew.-% Hydroxypropylstärkephosphat, und
c) 0,5 Gew.-% bis 2,5 Gew.-% Sorbitanmonostearat,
enthält und der Anteil der Ölphase der Emulsion von mehr als 5 Gew.-% bis 25 Gew.-% beträgt.

Eine bevorzugte Ausführungsform der Erfindung ist dadurch gekennzeichnet, dass die O/W-Emulsion, bezogen auf das Gesamtgewicht der Emulsion,
a) 0,1 Gew.-% bis 1,0 Gew.-% Natriumcetylsulfat, Natriumstearylsulfat und/oder Natriumcetearylsulfat,
b) 0,5 Gew.-% bis 3,0 Gew.-% Hydroxypropylstärkephosphat, und
c) 0,5 Gew.-% bis 2,5 Gew.-% Glycerinmonostearat und/oder Sorbitanmonostearat, enthält und der Anteil der Ölphase der Emulsion von mehr als 5 Gew.-% bis 25 Gew.-% beträgt.

Eine weitere bevorzugte Ausführungsform der Erfindung ist dadurch gekennzeichnet, dass die O/W-Emulsion, bezogen auf das Gesamtgewicht der Emulsion,
a) 0,1 Gew.-% bis 1,0 Gew.-% Natriumcetylsulfat, Natriumstearylsulfat und/oder Natriumcetearylsulfat,
b) 0,5 Gew.-% bis 3,0 Gew.-% Hydroxypropylstärkephosphat, und
c) 0,5 Gew.-% bis 2,5 Gew.-% Glycerinmonostearat,
enthält und der Anteil der Ölphase der Emulsion von mehr als 5 Gew.-% bis 25 Gew.-% beträgt.

Eine bevorzugte Ausführungsform der Erfindung ist dadurch gekennzeichnet, dass die O/W-Emulsion, bezogen auf das Gesamtgewicht der Emulsion,
a) 0,1 Gew.-% bis 1,0 Gew.-% Natriumcetylsulfat, Natriumstearylsulfat und/oder Natriumcetearylsulfat,
b) 0,5 Gew.-% bis 3,0 Gew.-% Hydroxypropylstärkephosphat, und
c) 0,5 Gew.-% bis 2,5 Gew.-% Sorbitanmonostearat,
enthält und der Anteil der Ölphase der Emulsion von mehr als 5 Gew.-% bis 25 Gew.-% beträgt.

Eine bevorzugte Ausführungsform der Erfindung ist dadurch gekennzeichnet, dass die O/W-Emulsion, bezogen auf das Gesamtgewicht der Emulsion,
a) 0,1 Gew.-% bis 1,0 Gew.-% mindestens eine Substanz gewählt aus der Gruppe der Alkalimetallalkylphosphate, dadurch gekennzeichnet, dass die Alkylgruppe jeweils 16 bis 18 Kohlenstoffatome aufweist, wobei die Substanz insbesondere bevorzugt Kaliumcetylphosphat ist,,
b) 0,5 Gew.-% bis 3,0 Gew.-% Hydroxypropylstärkephosphat, und
c) 0,5 Gew.-% bis 2,5 Gew.-% Glycerinmonostearat und/oder Sorbitanmonostearat, enthält und der Anteil der Ölphase der Emulsion von mehr als 5 Gew.-% bis 25 Gew.-% beträgt.

Eine weitere bevorzugte Ausführungsform der Erfindung ist dadurch gekennzeichnet, dass die O/W-Emulsion, bezogen auf das Gesamtgewicht der Emulsion,
a) 0,1 Gew.-% bis 1,0 Gew.-% mindestens eine Substanz gewählt aus der Gruppe der Alkalimetallalkylphosphate, dadurch gekennzeichnet, dass die Alkylgruppe jeweils 16 bis 18 Kohlenstoffatome aufweist, wobei die Substanz insbesondere bevorzugt Kaliumcetylphosphat ist,,
b) 0,5 Gew.-% bis 3,0 Gew.-% Hydroxypropylstärkephosphat, und
c) 0,5 Gew.-% bis 2,5 Gew.-% Glycerinmonostearat, enthält und der Anteil der Ölphase der Emulsion von mehr als 5 Gew.-% bis 25 Gew.-% beträgt.

Eine bevorzugte Ausführungsform der Erfindung ist dadurch gekennzeichnet, dass die O/W-Emulsion, bezogen auf das Gesamtgewicht der Emulsion,
a) 0,1 Gew.-% bis 1,0 Gew.-% mindestens eine Substanz gewählt aus der Gruppe der Alkalimetallalkylphosphate, dadurch gekennzeichnet, dass die Alkylgruppe jeweils 16 bis 18 Kohlenstoffatome aufweist, wobei die Substanz insbesondere bevorzugt Kaliumcetylphosphat ist,
b) 0,5 Gew.-% bis 3,0 Gew.-% Hydroxypropylstärkephosphat, und
c) 0,5 Gew.-% bis 2,5 Gew.-% Sorbitanmonostearat,
enthält und der Anteil der Ölphase der Emulsion von mehr als 5 Gew.-% bis 25 Gew.-% beträgt.

Es hat sich weiterhin überraschend gezeigt, dass die Temperaturstabilität der erfindungsgemäßen O/W-Emulsion weiter gesteigert werden konnte, indem der Emulsion ein oder mehrere Fettalkohole mit 14 bis 22 Kohlenstoffatomen zugesetzt werden. Der Gesamtanteil dieser Fettalkohole mit 14 bis 22 Kohlenstoffatomen beträgt vorteilhaft von 0,1 Gew.-% bis 5,0 Gew.-%, bevorzugt 0,2 Gew.-% bis 4,0 Gew.-% und insbesondere bevorzugt von 0,5 Gew.-% bis 3,0 Gew.-%, bezogen auf das Gesamtgewicht der Emulsion.

Insbesondere bevorzugt zu wählende Fettalkohole sind Cetylalkohol und/oder Stearylalkohol, wobei diese bevorzugt in den Anteilen von 0,1 Gew.-% bis 5,0 Gew.-%, bevorzugt 0,2 Gew.-% bis 4,0 Gew.-% und insbesondere bevorzugt von 0,5 Gew.-% bis 3,0 Gew.-%, bezogen auf das Gesamtgewicht der Emulsion, eingesetzt werden.

Vorteilhafte Ausführungsformen der vorliegenden Erfindung sind weiterhin dadurch gekennzeichnete, dass die O/W-Emulsion weitere Polysaccharid haltige Polymere enthält.

Vorteilhaft enthält die O/W-Emulsion dieser Ausführungsformen Tapiokastärke, wobei der Anteil von Tapiokastärke bevorzugt im Bereich von 0,5 Gew.-% bis 3,0 Gew.-% liegt, wobei sich die Gewichtsangaben auf das Gesamtgewicht der O/W-Emulsion beziehen.

Weiterhin enthält die O/W-Emulsion dieser Ausführungsformen vorteilhaft ein oder mehrere weitere Stärkephosphate, wobei der Gesamtanteil dieser zusätzlichen Stärkephosphate bevorzugt im Bereich von 0,5 Gew.-% bis 3,0 Gew.-% liegt, wobei sich die Gewichtsangaben auf das Gesamtgewicht der O/W-Emulsion beziehen. Unter dem Begriff "weiteren Stärkephosphaten" sind Stärkephosphate zu verstehen, welche von Hydroxypropylstärke verschieden sind.

Weiterhin enthält die O/W-Emulsion dieser Ausführungsformen vorteilhaft Xanthan und/oder ein modifiziertes Xanthan, wobei der Gesamtanteil dieser Polymere im Bereich von 0,05 Gew.-% bis 0,5 Gew.-% liegt, wobei sich die Gewichtsangaben auf das Gesamtgewicht der O/W-Emulsion beziehen.

Ferner enthält die O/W-Emulsion dieser Ausführungsformen vorteilhaft Carrageenan, wobei der Gesamtanteil von Carrageenan im Bereich von 0,05 Gew.-% bis 0,5 Gew.-% liegt, wobei sich die Gewichtsangaben auf das Gesamtgewicht der O/W-Emulsion beziehen.

Andere vorteilhafte Ausführungsformen der vorliegenden Erfindung sind ferner dadurch gekennzeichnet, dass diese neben Hydoxypropylstärkephosphat keine weiteren Stärken und Stärkederivate enthält.

Andere vorteilhafte Ausführungsformen der vorliegenden Erfindung sind ferner dadurch gekennzeichnet, dass diese keine Cellulosen oder Cellulosederivate enthält.

Andere vorteilhafte Ausführungsformen der vorliegenden Erfindung sind ferner dadurch gekennzeichnet, dass diese keine weiteren Polysaccharid-haltige Polymere enthält.

Die nachfolgend beschriebenen Merkmale der erfindungsgemäßen O/W-Emulsion beziehen sich auf die erfindungsgemäße O/W-Emulsion an sich, sowie jeweils auf die zuvor beschriebenen erfindungsgemäßen vorteilhaften Ausführungsformen.

Die erfindungsgemäße O/W-Emulsion enthält weiterhin vorteilhaft ein oder mehrere Lipidkomponenten, wie Wachse und Öle auf Basis von Kohlenwasserstoffen, gesättigte, ungesättige oder gehärtete Triglyceride, Dialkylether mit 12 bis 24 Kohlenstoffatomen und/oder die Ester aus einwertigen Alkoholen und Monocarbonsäuren, welche mindestens 10 Kohlenstoffatome aufweisen.

Wie bereits ausgeführt, ist es vorteilhaft, wenn die O/W-Emulsion der vorliegenden Erfindung ein oder mehrere verzweigte und/oder unverzweigte Kohlenwasserstoffe enthält, sofern diese nicht gasförmig unter Normalbedingungen sind. Vorteilhaft zu verwendende Kohlenwasserstoffe sind Paraffinum Liquidum, Isododecan, Isohexadecan, Isoeicosane, Squalan und Cera Microcristallina.

Die erfindungsgemäße O/W-Emulsion enthält vorteilhaft ein oder mehrere Triglycerinester, gewählt aus der Gruppe der synthetischen, halbsynthetischen und natürlichen Triglycerinester. Vorteilhafte natürliche Öle enthaltend derartige natürliche Triglycerinester sind Sonnenblumenöl (Helianthus Annuus Seed Oil), Rapsöl (Canola Oil), Sojaöl (Glycine Soja Oil), Olivenöl (Olea Europaea Fruit Oil), Mandelöl (Prunus amygdalus dulcis Oil), Avocadoöl (Persea Gratissima Oil), Walnussöl (Junglans Regia Seed Oil), Pfirsichkernöl (Prunus Persica Kernel Oil), Aprikosenkernöl (Prunus Armeniaca Kernel Oil), Sesamöl (Sesamum Indicum Seed Oil), Camelienöl (Camelia Oleifera/ Camelia Sasanqua), Nachtkerzenöl (Oenothera biennis), Macadamianussöl (Macadamia Intergrifolia Seed Oil), Diestelöl (Silybum Marianum Seed Oil), Weizenkeimöl (Triticum Vulgare Germ Oil), Palmkernöl (Elaeis guineensis Kernel Oil), Palmöl (Elaeis guineensis Oil), Traubenkernöl (Vitis Vinifera Seed Oil), Arganöl (Argania spinosa Seed Oil), Erdnussöl (Arachis hypogaea Oil), Kürbiskernöl (Cucurbita Pepo Seed Oil), Ricinusöl (Ricinus Communis Seed Oil), Reiskeimöl (Oryza Sativa Bran Oil), Vegetable Oil (Olus Oil) und Kokosöl.

Enthält die O/W-Emulsion ein oder mehrere der vorstehend genannten natürlichen Öle, so beträgt der Anteil dieser natürlichen Öle bevorzugt von 0,01 Gew.-% bis 3,0 Gew.-%, bezogen auf das Gesamtgewicht der O/W-Emulsion.

Zu den weiteren bevorzugten Triglycerinestern zählen unter anderen gehärtete Triglyceridfette, wie hydriertes Palmöl, hydriertes Kokosöl oder hydriertes Rizinusöl. Insbesondere ist der Einsatz von hydriertem Kokosöl (Hydrogenated Coco-Glycerides) bevorzugt, wobei der Anteil von hydriertem Kokosöl bevorzugt von 0,5 Gew.-% bis 3 Gew.-%, bezogen auf das Gesamtgewicht der Emulsion, beträgt.

Weiterhin ist es vorteilhaft, wenn die Emulsion Capryl-Caprinsäuretriglycerid enthält, wobei der Anteil von Capryl-Caprinsäuretriglycerid bevorzugt von 0,5 Gew.-% bis 5,0 Gew.-%, bezogen auf das Gesamtgewicht der O/W-Emulsion beträgt.

Ferner ist es vorteilhaft im Sinne der vorliegenden Erfindung, wenn die O/W-Emulsion der vorliegenden Erfindung ein oder mehrere Dialkylether mit 12 bis 24 Kohlenstoffatomen enthält, wobei bevorzugt Dicaprylylether enthalten ist. Enthält die O/W-Emulsion Dicaprylylether, so beträgt der Anteil von Dicaprylylether bevorzugt von 0,5 Gew.-% bis 3 Gew.-%, bezogen auf das Gesamtgewicht der O/W-Emulsion.

Weiterhin ist es vorteilhaft im Sinne der vorliegenden Erfindung, wenn die O/W-Emulsion der vorliegenden Erfindung ein oder mehrere Ester aus einwertigen Alkoholen und Monocarbonsäuren enthält, wobei die Ester mindestens 10, bevorzugt mindestens 15 Kohlenstoffatome aufweisen. Vorteilhaft zu wählende Ester aus einwertigen Alkoholen und Monocarbonsäuren sind 2-Ethylhexylisostearat, Isotridecylisononanoat, 2-Ethylhexylcocoat, C₁₂₋₁₅-Alkylbenzoat, Isopropylmyristat und Isopropylpalmitat.

Es ist weiterhin vorteilhaft im Sinne der vorliegenden Erfindung, wenn die O/W-Emulsion Octyldodecanol enthält.

Weiterhin ist es erfindungsgemäß bevorzugt, wenn die O/W-Emulsion keine Silikonöle enthält. Silikonöle haben oftmals den Effekt, dass sich Emulsionen besonders leicht anfühlen und schnell einziehen. Überraschend hat sich gezeigt, dass die erfindungsgemäße Emulsion auch ohne die Gegenwart von Silikonölen besonders schnell nach dem Auftragen auf die Haut einzieht.

Es ist darüber hinaus erfindungsgemäß von Vorteil, wenn die O/W-Emulsion Propylenglycol, Butylenglycol, 2-Methylpropan-1,3-diol, 1,2-Pentandiol, 1,2-Hexandiol, 1,2-Octandiol Glycerylcaprylate und/oder 1,2-Decandiol enthält.

Weiterhin ist es ebenfalls vorteilhaft im Sinne der vorliegenden Erfindung, wenn die O/W-Emulsion Phenoxyethanol, Methylparaben, Ethylparaben, Dehydracetsäure und/oder Ethylhexylglycerin enthält.

Enthält die O/W-Emulsion Phenoxyethanol, so ist es bevorzugt, wenn der Anteil an Phenoxyethanol von 0,1 Gew.-% bis 1,0 Gew.-% beträgt, bezogen auf das Gesamtgewicht der O/W-Emulsion.

Enthält die O/W-Emulsion Methylparaben und/oder Ethylparaben, so ist es bevorzugt, wenn der Gesamtanteil an Methylparaben und/oder Ethylparaben von 0,01 Gew.-% bis 0,5 Gew.-% beträgt, bezogen auf das Gesamtgewicht der O/W-Emulsion.

Darüber hinaus ist es vorteilhaft, wenn Ausführungsformer der Erfindung dadurch gekennzeichnet sind, dass diese Ethanol enthalten. Ist Ethanol in der O/W-Emulsion enthalten, so beträgt der Anteil von Ethanol bevorzugt von 0,5 Gew.-% bis 10 Gew.-%, bezogen auf das Gesamtgewicht der O/W-Emulsion.

Ferner ist die erfindungsgemäße O/W-Emulsion bevorzugt dadurch gekennzeichnet, dass diese Glycerin in einem Anteil von 0,5 Gew.-% bis 15% Gew.-% , bezogen auf das Gesamtgewicht der O/W-Emulsion, enthält.

Weiterhin, ist es bevorzugt, wenn die O/W-Emulsion neben den erfindungsgemäßen Emulgatoren und Tensiden, weitere Tenside und/oder Emulgatoren nur in einem Maximalanteil von 2 Gew.-%, bevorzugt in einem Maximalanteil von 1 Gew.-% und insbesondere bevorzugt keine weiteren Emulgatoren und Tenside enthält, wobei sich die Angaben auf das Gesamtgewicht der Emulsion beziehen.

Nicht zuletzt sind erfindungsgemäß vorteilhafte Ausführungsformen dadurch gekennzeichnet, dass die O/W-Emulsion einen oder mehrere Wirkstoffe gewählt aus der Gruppe der Verbindungen Glycyrrhetinsäure, Harnstoff, Arctiin, Folsäure, Coenzym Q10 (Ubiquinon), alpha-Glucosylrutin, Carnitin, Carnosin, Coffein, natürliche und/oder synthetische Isoflavonoide, Glycerylglucose, Kreatin, Kreatinin, Taurin, Tocopherol, Tocopherolacetat, Vitamin C, Vitamin C phosphate, Vitamin C palmitate, Niacinamide, Vitamin A palmitate, Panthenol, Licochalcon A, Rucinol, N-[(2,4-Dihydroxyphenyl)thiazol-2-yl]isobutyramide, Honociol und Magnolol (auch als Bestandteil von Magnolia-Extrakten), Hyaluronsäure und/oder Silymarin (Mariendistelextrakt) enthält.

Ferner sind erfindungsgemäß vorteilhafte O/W-Emulsionen dadurch gekennzeichnet, dass diese Wasser in einem Anteil von 60 Gew.-% bis 95 Gew.-% und bevorzugt von 70 Gew.-% bis 90 Gew.-%, bezogen auf das Gesamtgewicht der Emulsion enthalten.

Weiterhin weisen erfindungsgemäß vorteilhafte O/W-Emulsionen eine Viskosität von 4000 mPa·s bis 10000 mPa·s auf. Wird in dieser Offenbarung von Viskosität gesprochen, so beziehen sich alle Werte auf eine Messung bei 25°C im 150 ml Rollrandglas mittels Rheomat R 123 von der Firma proRheo. Der Rheomat R 123 der Firma proRheo GmbH ist ein Rotationsviskosimeter, d. h. ein Messkörper rotiert in der zu vermessenden Substanz. Es wird die Kraft gemessen, die benötigt wird, um den Messkörper in der Probe mit einer vorgegebenen Drehzahl rotieren zu lassen. Aus diesem Drehmoment, der Drehzahl des Messkörpers und den geometrischen Abmessungen des verwendeten Messystems wird die Viskosität berechnet. Als Messkörper wird der Messkörper Nr.1 (Artikelnr. 200 0191), Drehzahl Bereich 62,5 min⁻¹, verwendet. Alle Messungen zur Viskosität erfolgen immer 24h nach Herstellung der O/W-Emulsion.

### Vergleichsversuche und Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

In der nachfolgenden Tabellen werden die Beispielrezepturen Bsp.1 bis Bsp.13 aufgeführt, wobei nur die Beispielrezeptur Bsp.4 bis 7 und 10 bis 13 die erfindungsgemäße O/W-Emulsionen offenbaren. Die weiteren Beispielrezepturen sind nicht gemäß der Erfindung. Im Vergleich der erfindungsgemäßen und nicht erfindungsgemäßen O/W-Emulsionen zeigt sich, dass die erfindungsgemäßen O/W-Emulsionen keine Phasentrennung zeigen und so bei erhöhten Temperaturen deutlich stabiler sind.

Die O/W-Emulsionen werden hergestellt, indem Phase A und Phase C auf 75°C aufgeheizt werden bis sich alle Komponenten gelöst haben. Anschließend werden die Phasen vermischt. Die Mischung wird homogenisiert und anschließend unter Rühren auf Raumtemperatur abgekühlt. Phase B wird in 60mL Wasser (von Phase A abgenommen) eingerührt und nach Phase D zur Emulsion dazugegeben. Die Emulsion wird dann erneut homogenisiert.

| **Phase** | **Inhaltstoffe** | **Bsp.1** | **Bsp.2** | **Bsp.3** | **Bsp.4** |
|---|---|---|---|---|---|
| A | Glycerin | 5 | 5 | 5 | 5 |
| | Aqua | Ad 100 | Ad 100 | Ad 100 | Ad 100 |
| | Sodium cetearylsulfate | 0,2 | 0,2 | 0,2 | 0,2 |
| B | Hydroxypropyl starch phosphate' | - | - | 2,5 | 2,5 |
| | Tapioca starch | - | - | - | - |
| | Distarch phosphate | | | | |
| C | Cetyl alcohol | 2 | 2 | 2 | 2 |
| | Hydrogenated coco-glycerides | 2 | 2 | 2 | 2 |
| | Dicaprylyl ether | 3,8 | 3,8 | 3,8 | 3,8 |
| | Caprylic/capric triglyceride | 4 | 4 | 4 | 4 |
| | Glyceryl stearate | - | 1 | - | 1 |
| D | Phenoxyethanol | 0,8 | 0,8 | 0,8 | 0,8 |
| | Trisodium EDTA | 0,2 | 0,2 | 0,2 | 0,2 |
| | Methylparabene | 0,3 | 0,3 | 0,3 | 0,3 |

| **Lagerung** | | | | | |
|---|---|---|---|---|---|
| bei 40°C für 7 Tage | | Phasentrennung | Phasentrennung | Phasentrennung | Stabil |
| bei 50°C für 7 Tage | | Phasentrennung | Phasentrennung | Phasentrennung | Stabil |
| Eigenschaften bei Auftragung auf der Haut | | | | | Keine Rückstände, schnelle Absorption auf der Haut |

| | | | | | |
|---|---|---|---|---|---|
| ¹ Handelsprodukt: C*HiForm A 12747 von Cargill | | | | | |

| **Phase** | **Inhaltstoffe** | **Bsp.5** | **Bsp.6** | **Bsp.7** | **Bsp.8** | **Bsp.9** |
|---|---|---|---|---|---|---|
| A | Glycerin | 5 | 5 | 5 | 5 | 5 |
| | Aqua | Ad 100 | Ad 100 | Ad 100 | Ad 100 | Ad 100 |
| | Sodium cetearylsulfate | 1,5 | 0,2 | 0,2 | 0,2 | 0,2 |
| B | Hydroxypropyl starch phosphate' | 2,5 | 5,5 | 2,5 | - | - |
| | Tapioca starch | - | - | - | 2,5 | - |
| | Distarch phosphate | - | - | - | - | 2,5 |
| C | Cetyl alcohol | 2 | 2 | 2 | 2 | 2 |
| | Hydrogenated coco-glycerides | 2 | 2 | 2 | 2 | 2 |
| | Dicaprylyl ether | 3,8 | 3,8 | 3,8 | 3,8 | 3,8 |
| | Caprylic/capric triglyceride | 4 | 4 | 4 | 4 | 4 |
| | Glyceryl stearate | 1 | 1 | 3,5 | 1 | 1 |
| D | Phenoxyethanol | 0,8 | 0,8 | 0,8 | 0,8 | 0,8 |
| | Trisodium EDTA | 0,2 | 0,2 | 0,2 | 0,2 | 0,2 |
| | Methylparabene | 0,3 | 0,3 | 0,3 | 0,3 | 0,3 |

| **Lagerung** | | | | | | |
|---|---|---|---|---|---|---|
| bei 40°C für 7 Tage | | Stabil | Stabil | Stabil | Phasentrennung | Phasentrennung |
| bei 50°C für 7 Tage | | Stabil | Stabil | Stabil | Phasentrennung | Phasentrennung |
| Eigenschaften bei Auftragung auf der Haut | | deutliche Rückstände, zieht langsam ein | deutliche Rückstände, zieht langsam ein | deutliche Rückstände, zieht langsam ein | | |

| | | | | | | |
|---|---|---|---|---|---|---|
| ¹ Handelsprodukt: C*HiForm A 12747 von Cargill | | | | | | |

In den nachfolgenden Beispielen werden die in der nachfolgenden Tabelle aufgeführt Substanzen jeweils eingesetzt.

| | |
|---|---|
| Substanz A | Natriumstearoylglutamat, Natriumcetylstearylsulfat oder Kaliumcetylphosphat |
| Substanz B | Glycerinmonostearat oder Sorbitanmonostearate |

Das heißt, dass wenn eine Beispielszubereitung den Inhaltsstoff Substanz A enthält, so ist diese Beispielzubereitung einmal mit Natriumstearoylglutamat, einmal mit Natriumcetylstearylsulfat und einmal mit Kaliumcetylphosphat dargestellt. Analoges gilt für Substanz B.

Formulierungen, welche ausschließlich Natriumstearoylglutamat als Substanz A enthalten, sind nicht Teil der Erfindung.

| **Phase** | **Inhaltstoffe** | **Bsp.10** | **Bsp.11** | **Bsp.12** | **Bsp.13** |
|---|---|---|---|---|---|
| A | Glycerin | 5 | 5 | 5 | 5 |
| | Aqua | Ad 100 | Ad 100 | Ad 100 | Ad 100 |
| | Substanz A | 0,2 | 1,5 | 0,2 | 0,2 |
| B | Hydroxypropyl starch phosphate' | 2,5 | 2,5 | 5,5 | 2,5 |
| C | Cetyl alcohol | 2 | 2 | 2 | 2 |
| | Hydrogenated coco-glycerides | 2 | 2 | 2 | 2 |
| | Dicaprylyl ether | 3,8 | 3,8 | 3,8 | 3,8 |
| | Caprylic/capric triglyceride | 4 | 4 | 4 | 4 |
| | Substanz B | 1 | 1 | 1 | 3,5 |
| D | Phenoxyethanol | 0,8 | 0,8 | 0,8 | 0,8 |
| | Trisodium EDTA | 0,2 | 0,2 | 0,2 | 0,2 |
| | Methylparabene | 0,3 | 0,3 | 0,3 | 0,3 |

| | | | | | |
|---|---|---|---|---|---|
| ¹ Handelsprodukt: C*HiForm A 12747 von Cargill | | | | | |

| **Beispiel A (nicht erfindungsgemäß)** | **Gew.-%** |
|---|---|
| O/W-Creme | |
| Natriumstearoylglutamat | 0,3 |
| Glycerinmonostearat | 3 |
| C₁₂₋₁₅ Alkylbenzoat | 2 |
| Dicaprylylether | 2 |
| Octyldodecanol | 2 |
| Caprylsäure/Caprinsäuretriglyceride | 1 |
| Joojobaöl | 0,5 |
| Tocopherylacetat | 0,3 |
| Natriumascorbylphosphat | 0,1 |
| Phenoxyethanol | 0,7 |
| Hydroxypropyl starch phosphate | 3 |
| Octandiol | 0,3 |
| Benzethonium chloride | 0,05 |
| Pentylenglycol | 2 |
| Glycerin | 7 |
| Füllstoffe/ Additive (TiO₂, BHT) | 0,5 |
| Parfüm | q.s. |
| Wasser | ad 100 |

| **Beispiel B** | **Gew.-%** |
|---|---|
| O/W-Tagescreme | |
| Natriumcetylstearylsulfat | 0,2 |
| Sorbitanmonostearat | 2 |
| Cetearylalkohol | 1 |
| C₁₂₋₁₅ Alkylbenzoat | 3 |
| Caprylsäure/Caprinsäuretriglyceride | 2 |
| Dicaprylylcarbonat | 2 |
| Octyldodecanol | 1 |
| Macadamiaöl | 0,5 |
| Octocrylene | 2 |
| Ethylhexylmethoxycinnamat | 3 |
| Octylsalicylat | 2 |
| TiO₂ | 1 |
| Glycyrrhetinsäure | 0.1 |
| Folsäure | 0,05 |
| Phenoxyethanol | 0,8 |
| Octopirox | 0,1 |
| Hexandiol | 1 |
| EDTA | 0,2 |
| Hydroxypropyl starch phosphate | 2,5 |
| Glycerin | 7 |
| Additive (Xanthan Gum, BHT) | 0,3 |
| Parfüm | q.s. |
| Wasser | ad 100 |

| | |
|---|---|
| pH 7,0 | |

| **Beispiel C (nicht erfindungsgemäß)** | **Gew.-%** |
|---|---|
| O/W-Tagescreme | |
| Natriumstearoylglutamat | 0,3 |
| Sorbitanmonostearat | 1 |
| Cetearylalkohol | 2 |
| Isopropylpalmitate | 3 |
| Ethylhexylstearat | 1 |
| Hydrierte Kokosfettglyceride (Hydrogenated Coco Glycerides) | 1 |
| C₁₂₋₁₅ Alkylbenzoat | 2 |
| Dibutyladipate | 1 |
| Tocophol | 0,1 |
| Coenzym Q10 (Ubiquinon) | 0,05 |
| Milchsäure Natriumsalz | 0,3 |
| Methylpropandiol | 2 |
| Phenoxyethanol | 0,5 |
| Benzylalkohol | 0,2 |
| Hydroxypropyl starch phosphate | 2 |
| Hyaluronsäure, Natriumsalz | 0,1 |
| NaOH_{aq} | q.s. |
| Glycerin | 8 |
| Additive (EDTA, Carrageenan) | 0,6 |
| Parfüm | q.s. |
| Wasser | ad 100 |

| | |
|---|---|
| pH 7,0 | |

| **Beispiel D** | **Gew.-%** |
|---|---|
| O/W Creme | |
| Natriumcetylstearylsulfat | 0,3 |
| Sorbitanmonostearat | 2 |
| Cetylalkohol | 1 |
| C₁₂₋₁₅ Alkylbenzoat | 2 |
| Sheabutter | 0,5 |
| Caprylsäure/Caprinsäuretriglyceride | 3 |
| Dicaprylylcarbonat | 2 |
| Dicaprylylether | 1 |
| Ethylhexylcocoate | 1 |
| Isohexadecan | 1 |
| Citronensäure Natriumsalz | qs. |
| Phenoxyethanol | 0,8 |
| Dipropylenglycol | 1 |
| Benzylalkohol | 0,3 |
| Glycyrrhiza inflata Extrakt (Licochalcone A) | 0,1 |
| Ethylhexylglycerin | 0,4 |
| Hydroxypropyl starch phosphate | 2 |
| EDTA | 0,2 |
| Ethanol | 1 |
| Glycerin | 5 |
| Additive (EDTA, Locust bean gum) | 0,6 |
| NaOH_{aq} | q.s. |
| Parfüm | q.s. |
| Wasser | ad 100 |

| | |
|---|---|
| pH 5,2 | |

| **Beispiel E** | **Gew.-%** |
|---|---|
| O/W-Creme | |
| Kaliumcetylphosphat | 1 |
| Glycerylstearate | 2 |
| Cetearylalkohol | 1 |
| Caprylsäure/Caprinsäuretriglyceride | 2 |
| C₁₂₋₁₅ Alkylbenzoat | 2 |
| Sonnenblumenöl | 0,5 |
| Cocosglyceride | 2 |
| Octyldodecanol | 1 |
| Triisostearin | 1 |
| Cetearylisononanoate | 1 |
| p-Hydroxybenzoesäuremethylester (Paraben) | 0,2 |
| Hydroxypropyl starch phosphate | 1,5 |
| Octandiol | 0,2 |
| 4-Hydroxyacetophenon | 0,2 |
| Glycerin | 5 |
| Panthenol | 1 |
| Additive (Hydroxypropylmethylcellulose, BHT) | 0,5 |
| Parfüm | q.s. |
| Wasser | ad 100 |

## Patentansprüche

1. Acrylatfreie kosmetische O/W-Emulsion enthaltend
a) mindestens eine Substanz gewählt aus der Gruppe der Alkalimetallalkylsulfate und der Alkalimetallalkylphosphate, wobei die Alkylgruppe jeweils 16 bis 18 Kohlenstoffatome aufweist,
b) Hydroxypropylstärkephosphat, und
c) Glycerinmonostearat und/oder Sorbitanmonostearat.

2. Verwendung einer Mischung aus
a) mindestens eine Substanz gewählt aus der Gruppe der Alkalimetallalkylsulfate und der Alkalimetallalkylphosphate, wobei die Alkylgruppe jeweils 16 bis 18 Kohlenstoffatome aufweist,
b) Hydroxypropylstärkephosphat, und
c) Glycerinmonostearat und/oder Sorbitanmonostearat.
zur Verhinderung einer Phasentrennung in einer acrylatfreien O/W-Emulsion.

3. Emulsion oder Verwendung nach einem der vorhergehenden Ansprüche **dadurch gekennzeichnet, dass** als Substanz a) Natriumcetylsulfat, Natriumstearylsulfat und/oder Natriumcetearylsulfat enthalten sind.

4. Emulsion oder Verwendung nach Anspruch 1 und/oder Anspruch 2 **dadurch gekennzeichnet, dass** die Substanz a) gewählt wird aus der Gruppe der Alkalimetallalkylphosphate, **dadurch gekennzeichnet, dass** die Alkylgruppe jeweils 16 bis 18 Kohlenstoffatome aufweist, wobei die Substanz Kaliumcetylphosphat bevorzugt ist.

5. Emulsion oder Verwendung nach einem der vorhergehenden Ansprüche **dadurch gekennzeichnet, dass** der Gesamtanteil der Substanzen a) von 0,01 Gew.-% bis 5,0 Gew.-%, mehr bevorzugt von 0,05 Gew.-% bis 2,0 Gew.-% und am meisten bevorzugt von 0,1 Gew.-% bis 1,5 Gew.-%, bezogen auf das Gesamtgewicht der Emulsion, beträgt.

6. Emulsion oder Verwendung nach einem der vorhergehenden Ansprüche **dadurch gekennzeichnet, dass** der Anteil von Hydroxypropylstärkephosphat von 0,1 Gew.-% bis 10 Gew.-%, bevorzugt von 0,2 Gew.-% bis 5,0 Gew.-% und insbesondere bevorzugt von 0,5 Gew.-% bis 3 Gew.-%, bezogen auf das Gesamtgewicht der Emulsion, beträgt.

7. Emulsion oder Verwendung nach einem der vorhergehenden Ansprüche **dadurch gekennzeichnet, dass** das Hydroxypropylstärkephosphat ein Veresterungsprodukt basierend auf Maisstärke ist.

8. Emulsion oder Verwendung nach einem der vorhergehenden Ansprüche **dadurch gekennzeichnet, dass** der Anteil von Glycerinmonostearat und/oder Sorbitanmonostearat von 0,1 Gew.-% bis 5,0 Gew.-%, bevorzugt 0,2 Gew.-% bis 3,0 Gew.-% und insbesondere bevorzugt von 0,5 Gew.-% bis 2,5 Gew.-%,beträgt, wobei sich die Angaben auf das Gesamtgewicht der Emulsion beziehen.

9. Emulsion oder Verwendung nach einem der vorhergehenden Ansprüche **dadurch gekennzeichnet, dass** der Anteil der Ölphase der Emulsion von mehr als 3 Gew.-% bis 30 Gew.-%, bevorzugt von mehr als 4 Gew.-% bis 28 Gew.-% und insbesondere bevorzugt von mehr als 5 Gew.-% bis 25 Gew.-%, bezogen auf das Gesamtgewicht der Emulsion, beträgt.

10. Emulsion oder Verwendung nach einem der vorhergehenden Ansprüche **dadurch gekennzeichnet, dass** die Emulsion ein oder mehrere Fettalkohole mit 14 bis 22 Kohlenstoffatomen enthält und der Gesamtanteil dieser Fettalkohol vorteilhaft von 0,1 Gew.-% bis 5,0 Gew.-%, bevorzugt 0,2 Gew.-% bis 4,0 Gew.-% und insbesondere bevorzugt von 0,5 Gew.-% bis 3,0 Gew.-%, bezogen auf das Gesamtgewicht der Emulsion, beträgt.

11. Emulsion oder Verwendung nach einem der vorhergehenden Ansprüche **dadurch gekennzeichnet, dass** die O/W-Emulsionen Wachse und Öle auf Basis von Kohlenwasserstoffen; gesättigte, ungesättige oder gehärtete Triglyceride; Dialkylether mit 12 bis 24 Kohlenstoffatomen und/oder die Ester aus einwertigen Alkoholen und Monocarbonsäuren, welche mindestens 10 Kohlenstoffatome aufweisen, enthält.

12. Emulsion oder Verwendung nach einem der vorhergehenden Ansprüche **dadurch gekennzeichnet, dass** die Emulsion keine Silikonöle enthält.

13. Emulsion oder Verwendung nach einem der vorhergehenden Ansprüche **dadurch gekennzeichnet, dass** weiteren Tenside und/oder Emulgatoren nur in einem Maximalanteil von 2 Gew.-%, bevorzugt in einem Maximalanteil von 1 Gew.-% und insbesondere bevorzugt keine weiteren Emulgatoren und Tenside enthält, wobei sich die Angaben auf das Gesamtgewicht der Emulsion beziehen.

14. Emulsion oder Verwendung nach einem der vorhergehenden Ansprüche **dadurch gekennzeichnet, dass** die Emulsion Wasser in einem Anteil von 60 Gew.-% bis 95 Gew.-% und bevorzugt von 70 Gew.-% bis 90 Gew.-%, bezogen auf das Gesamtgewicht der Emulsion, enthält.

## Claims

1. Acrylate-free cosmetic O/W emulsion comprising
a) at least one substance selected from the group comprising alkali metal alkyl sulfates and alkali metal alkyl phosphates, wherein the alkyl group has in each case 16 to 18 carbon atoms,
b) hydroxypropyl starch phosphate, and
c) glycerol monostearate and/or sorbitan monostearate.

2. Use of a mixture composed of
a) at least one substance selected from the group comprising alkali metal alkyl sulfates and alkali metal alkyl phosphates, wherein the alkyl group has in each case 16 to 18 carbon atoms,
b) hydroxypropyl starch phosphate, and
c) glycerol monostearate and/or sorbitan monostearate to prevent phase separation in an acrylate-free O/W emulsion.

3. Emulsion or use according to either of the preceding claims, **characterized in that** sodium cetyl sulfate, sodium stearyl sulfate and/or sodium cetearyl sulfate are present as substance a).

4. Emulsion or use according to Claim 1 and/or Claim 2, **characterized in that** substance a) is selected from the group of alkali metal alkyl phosphates, **characterized in that** the alkyl group has in each case 16 to 18 carbon atoms, wherein the substance is preferably potassium cetyl phosphate.

5. Emulsion or use according to any of the preceding claims, **characterized in that** the total proportion of the substances a) is from 0.01% by weight to 5.0% by weight, more preferably from 0.05% by weight to 2.0% by weight and most preferably from 0.1% by weight to 1.5% by weight, based on the total weight of the emulsion.

6. Emulsion or use according to any of the preceding claims, **characterized in that** the proportion of hydroxypropyl starch phosphate is from 0.1% by weight to 10% by weight, preferably from 0.2% by weight to 5.0% by weight and particularly preferably from 0.5% by weight to 3% by weight, based on the total weight of the emulsion.

7. Emulsion or use according to any of the preceding claims, **characterized in that** the hydroxypropyl starch phosphate is an esterification product based on corn starch.

8. Emulsion or use according to any of the preceding claims, **characterized in that** the proportion of glycerol monostearate and/or sorbitan monostearate is from 0.1% by weight to 5.0% by weight, preferably 0.2% by weight to 3.0% by weight and particularly preferably from 0.5% by weight to 2.5% by weight, wherein the figures refer to the total weight of the emulsion.

9. Emulsion or use according to any of the preceding claims, **characterized in that** the proportion of the oil phase of the emulsion is from more than 3% by weight to 30% by weight, preferably from more than 4% by weight to 28% by weight and particularly preferably from more than 5% by weight to 25%) by weight, based on the total weight of the emulsion.

10. Emulsion or use according to any of the preceding claims, **characterized in that** the emulsion comprises one or more fatty alcohols having 14 to 22 carbon atoms and the total proportion of said fatty alcohols is advantageously from 0.1% by weight to 5.0% by weight, preferably 0.2% by weight to 4.0% by weight and particularly preferably from 0.5% by weight to 3.0% by weight, based on the total weight of the emulsion.

11. Emulsion or use according to any of the preceding claims, **characterized in that** the O/W emulsion comprises waxes and oils based on hydrocarbons; saturated, unsaturated or hardened triglycerides; dialkyl ethers having 12 to 24 carbon atoms and/or esters of monohydric alcohols and monocarboxylic acids having at least 10 carbon atoms.

12. Emulsion or use according to any of the preceding claims, **characterized in that** the emulsion does not comprise silicone oils.

13. Emulsion or use according to any of the preceding claims, **characterized in that** comprises further surfactants and/or emulsifiers only at a maximum proportion of 2% by weight, preferably at a maximum proportion of 1% by weight, and particularly preferably does not comprise any further emulsifiers and surfactants, wherein the figures refer to the total weight of the emulsion.

14. Emulsion or use according to any of the preceding claims, **characterized in that** the emulsion comprises water in a proportion of 60% by weight to 95% by weight and preferably from 70% by weight to 90% by weight, based on the total weight of the emulsion.

## Revendications

1. Émulsion cosmétique H/E sans acrylate, contenant
a) au moins une substance choisie dans le groupe des alkylsulfates de métaux alcalins et des alkylphosphates de métaux alcalins, dans lesquels le groupe alkyle comporte chaque fois de 16 à 18 atomes de carbone,
b= du phosphate d'hydroxypropylamidon, et
c) du monostéarate de glycérol et/ou du monostéarate de sorbitane.

2. Utilisation d'un mélange de
a) au moins une substance choisie dans le groupe des alkylsulfates de métaux alcalins et des alkylphosphates de métaux alcalins, dans lesquels le groupe alkyle comporte chaque fois de 16 à 18 atomes de carbone,
b= du phosphate d'hydroxypropylamidon, et
c) du monostéarate de glycérol et/ou du monostéarate de sorbitane.
pour empêcher une séparation de phases dans une émulsion H/E sans acrylate.

3. Émulsion ou utilisation selon l'une quelconque des revendications précédentes, **caractérisée en ce qu'**en tant que substance a) sont contenus du cétylsulfate de sodium, du stéarylsulfate de sodium et/ou du cétéarylsulfate de sodium.

4. Émulsion ou utilisation selon la revendication 1 et/ou la revendication 2, **caractérisée en ce que** la substance a) est choisie dans le groupe des alkylphosphates de métaux alcalins **caractérisés en ce que** le groupe alkyle comporte chaque fois de 16 à 18 atomes de carbone, la substance cétylphosphate de potassium étant préférée.

5. Émulsion ou utilisation selon l'une quelconque des revendications précédentes, **caractérisée en ce que** la proportion totale des substances a) vaut de 0,01 % en poids à 5,0 % en poids, de façon plus particulièrement préférée de 0,05 % en poids à 2,0 % en poids et de façon tout particulièrement préférée de 0,1 % en poids à 1,5 % en poids, par rapport au poids total de l'émulsion.

6. Émulsion ou utilisation selon l'une quelconque des revendications précédentes, **caractérisée en ce que** la proportion totale des substances a) vaut de 0,1 % en poids à 10 % en poids, de façon plus particulièrement préférée de 0,2 % en poids à 5,0 % en poids et de façon tout particulièrement préférée de 0,5 % en poids à 3 % en poids, par rapport au poids total de l'émulsion.

7. Émulsion ou utilisation selon lune quelconque des revendications précédentes, **caractérisée en ce que** le phosphate d'hydroxypropylamidon est un produit d'estérification à base d'amidon de maïs.

8. Émulsion ou utilisation selon l'une quelconque des revendications précédentes, **caractérisée en ce que** la proportion de monostéarate de glycérol et/ou monostéarate de sorbitane vaut de 0,1 % en poids à 5,0 % en poids, de préférence 0,2 % en poids à 3,0 % en poids et de façon particulièrement préférée de 0,5 % en poids à 2,5 % en poids, les données se rapportant au poids total de l'émulsion.

9. Émulsion ou utilisation selon l'une quelconque des revendications précédentes, **caractérisée en ce que** la proportion de la phase huileuse de l'émulsion vaut de 3 % en poids à 30 % en poids, de préférence de plus de 4 % en poids à 28 % en poids et de façon particulièrement préférée de plus de 5 % en poids à 25 % en poids, par rapport au poids total de l'émulsion.

10. Émulsion ou utilisation selon l'une quelconque des revendications précédentes, **caractérisée en ce que** l'émulsion contient un ou plusieurs alcools gras ayant de 14 à 22 atomes de carbone et la proportion totale de cet alcool gras vaut avantageusement de 0,1 % en poids à 5,0 % en poids, de préférence 0,2 % en poids à 4,0 % en poids et de façon particulièrement préférée de 0,5 % en poids à 3,0 % en poids, par rapport au poids total de l'émulsion.

11. Émulsion ou utilisation selon l'une quelconque des revendications précédentes, **caractérisée en ce que** les émulsions H/E contiennent des cires et huiles à base d'hydrocarbures ; des triglycérides saturés, insaturés ou hydrogénés ; des éthers dialkyliques ayant de 12 à 24 atomes de carbone et/ou les esters d'alcools monohydriques et d'acides monocarboxyliques, qui comportent au moins 10 atomes de carbone.

12. Émulsion ou utilisation selon l'une quelconque des revendications précédentes, **caractérisée en ce que** l'émulsion ne contient pas d'huiles de silicone.

13. Émulsion ou utilisation selon l'une quelconque des revendications précédentes, caractérisée en qu'elle ne contient des tensioactifs et/ou émulsifiants supplémentaires qu'en une proportion maximale de 2 % en poids, de préférence en une proportion maximale de 1 % en poids, et de façon particulièrement préférée ne contient pas d'émulsifiants et de tensioactifs supplémentaires, les données se rapportant au poids total de l'émulsion.

14. Émulsion ou utilisation selon l'une quelconque des revendications précédentes, **caractérisée en ce que** l'émulsion contient de l'eau en une proportion de 60 % en poids à 95 % en poids et de préférence de 70 % en poids à 90 % en poids, par rapport au poids total de l'émulsion.
